# EUROPEAN PATENT SPECIFICATION

(11) **EP 1 503 721 B1**
(45) Date of publication and mention of the grant of the patent: **06.09.2006**
(21) Application number: 03720446.8
(22) Date of filing: 09.04.2003
(51) Int. Cl.: A61K 8/34, A61K 8/368, A61Q 5/02, A61Q 5/06, A61Q 5/12

(54) **HAIR CARE COMPOSITIONS CONTAINING PHENOLIC STYLING AGENTS**
HAARPFLEGEMITTEL, DIE PHENOLISCHE HAARFORMUNGSMITTEL ENTHALTEN
COMPOSITIONS DE SOIN CAPILLAIRE CONTENANT DES AGENTS DE COIFFAGE PHENOLIQUES

(30) Priority: 15.05.2002 EP 02253409
(43) Date of publication of application: 09.02.2005
(73) Proprietor: UNILEVER N.V., 3013 AL Rotterdam (NL); UNILEVER PLC, London, Greater London EC4 4BQ (GB)
(72) Inventor: Khosdel, Ezat, Bebington, WIrral,2Merseyside CH63 3JW (GB); Ward, Sheila Anne, Southgate, London N14 4PW (GB)
(74) Representative: Tansley, Sally Elizabeth
(86) International application number: PCT/EP2003/003715
(87) International publication number: WO 2003/096996

(56) References cited:
- EP-A- 0 054 174
- EP-A- 0 437 114
- EP-A- 0 842 654
- DE-A- 2 062 923
- DE-A- 2 939 303
- DE-A- 3 115 537
- DE-B- 2 830 497
- DATABASE CA [Online] CHEMICAL ABSTRACTS SERVICE, COLUMBUS, OHIO, US; retrieved from STN Database accession no. 116:91137/DN, HCAPLUS XP002218054 & JP 03 246215 A (LION CORP.) 1 November 1991 (1991-11-01)
- PATENT ABSTRACTS OF JAPAN vol. 010, no. 276 (C-373), 10 September 1986 (1986-09-10) & JP 61 097212 A (ABE YUKAGAKU KENKYUSHO:KK), 15 May 1986 (1986-05-15)
- PATENT ABSTRACTS OF JAPAN vol. 018, no. 680 (C-1291), 21 December 1994 (1994-12-21) & JP 06 271428 A (POLA CHEM IND INC), 27 September 1994 (1994-09-27)
- DATABASE WPI Section Ch, Week 197851 Derwent Publications Ltd., London, GB; Class D21, AN 1978-92031A XP002218055 & JP 53 130443 A (SHISEIDO CO LTD), 14 November 1978 (1978-11-14)

## Description

### Field of the Invention

This invention relates to hair care compositions, in particular to hair care compositions that style the hair.

### Background and Prior Art

The desire to have the hair retain a particular shape or style is widely held. The most common approach for accomplishing styling of hair is the application of a composition to dampened hair, after shampooing and/or conditioning, or to dry, styled hair. These compositions provide temporary styling benefits and can readily be removed by water or shampooing. To date, the materials employed in hair care compositions to provide styling benefits have generally been natural or synthetic resins such as polymers of acrylate and polymers of acrylate with grafted silicone copolymers. Styling compositions are usually applied in the form of, sprays, mousses, gels and lotions.

Hair setting compositions comprising gallic acid or its features are described in EP 0 437 114 (Unilevel), JP 03 246 215 (Lion) JP 61 097212 (ABE Yakagaku) and DE 20 62 923 (l'Obal).

A method of setting hair has been described in US 4 452 261 (Bresak et al) involving the application to hair of a dialdehyde polysaccharide and a hydroxyarnmatic compound, on heating whist drying the hair these two compounds react together and so set the hair.

### Description of the Present invention

According to the present invention, there is provided a method of styling hair comprising the step of applying to the hair a non-colourant hair treatment composition comprising 0.1 to 3 wt% of a styling compound of formula 1 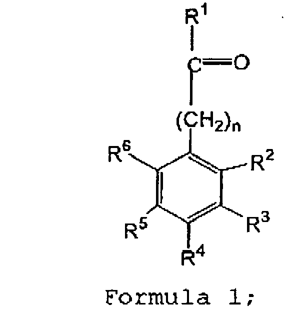
wherein n is equal to 0,
R¹ is a H, OH, alkyl, alkoxide group or salts thereof;
R², R³ , R⁴ , R⁵ and R⁶ are independently selected from OH, H, C₁-C₄ alkyl groups or mixtures thereof such that the total number of OH groups of R², R³, R⁴, R⁵ and R⁶ is 2, such that when R² is an OH group R⁵ is also an OH group;
in which either the composition comprises less than 2 wt% of a starch or starch derivative or the composition is free of compounds capable of reacting below 70°C to form covalent bonds with the compound of formula 1, when both compounds are applied to the hair.

In another aspect of the invention there is provided the use of a compound of formula 1 for styling hair.

### Detailed Description of the Invention

### Styling compound

Hair treatment compositions according to the invention, comprise at least one styling compound of formula 1 in order to provide improved hold to the hair. The styling compound according to the invention is non-polymeric.

In one embodiment of the invention, when applied to the hair and styled using normal temperatures (70°C or below) the compound of formula 1 does not react with any other compound in the composition to form a covalent bond.

It is thought the compound of formula 1 interacts with the hair via non-covalent interactions (secondary forces).
Preferred non-covalent interactions include H-bonding, electrostatic forces and II-stacking.

It is also highly preferable if R², R³, R⁴, R⁵ and R⁶ are either OH or H.

If R², R³, R⁴, R⁵ and R⁶ are equal to C₁-C₄ alkyl groups it is preferable if they are not branched, more preferably that they are methyl groups.

If R¹ is a salt it is preferable if it is a salt of an alkali metal (preferably sodium or potassium) or an amine. Mixtures of partially and fully neutralised systems can also be used.

Especially preferred are styling compounds having the following formulae:

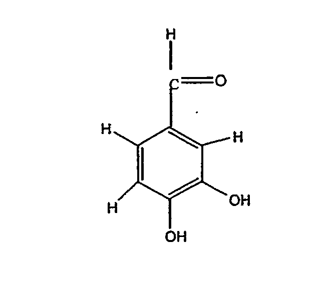

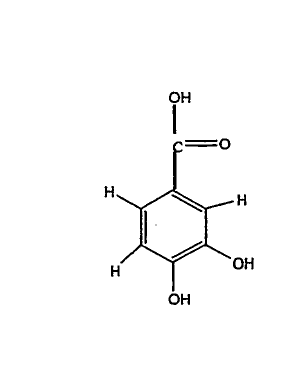

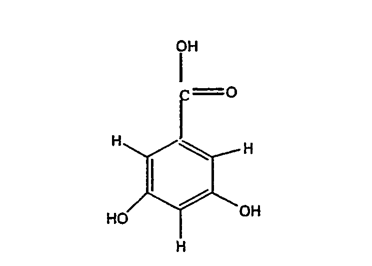

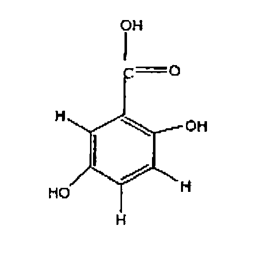

The level of this styling compound in the composition is 0.1 wt% to 3 wt% of the total composition.

### Product Type

Compositions of the present invention are formulated into hair care compositions, especially products with hair styling claims, but can also be formulated into a wide variety of product types, including mousses, gels, lotions, tonics, sprays, shampoos, conditioners, rinses, Compositions of the invention typically comprise a cosmetically acceptable diluent or carrier. Preferably, the compositions are for use in styling human hair and, more preferably, they are packaged and labelled as such.

Compositions of the invention are not formulated so as to be suitable for use in the laundering of clothes ie, they are not laundry compositions.

The carriers and additional components required to formulate such products vary with product type and can be routinely chosen by one skilled in the art. The following is a description of some of these carriers and additional components.

Hair care compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, of the composition. As used herein, the phrase "suitable for application to hair" means that the carrier does not damage or negatively affect the aesthetics of hair or cause irritation to the underlying skin.

In one aspect of the invention the composition does not contain greater than 2 wt% of a starch or starch derivative, preferably the composition contains less than 1 wt% of a starch or a starch derivative, more preferably the composition is free of starch or starch derivatives.

It is preferable if the compositions according to the invention comprise a buffer or pH adjuster. Preferred buffers or pH adjusters include weak acids and bases such glycine/sodium hydroxide, citric acid, lactic acid, succinic acid, acetic salt and salts thereof. Frequently a mixture of buffering system is used such as sodium citrate and citric acid.

Carriers suitable for use with hair care compositions of the present invention include, for example, those used in the formulation of hair sprays, mousses, tonics, gels, shampoos, conditioners, and rinses. The choice of appropriate carrier will depend on the particular product to be formulated. The carriers used herein can include a wide range of components conventionally used in hair care compositions. The carriers can contain a solvent to dissolve or disperse the styling compound being used, with water, the C₁-C₆ alcohols, lower alkyl acetate and mixtures thereof being preferred. The carriers can also contain a wide variety of additional materials such as acetone, hydrocarbons (such as isobutane, hexane, decene), halogenated hydrocarbons (such as Freons) and volatile silicones such as cyclomethicone.

When the hair care composition is a hair spray, tonic, gel, or mousse the preferred solvents include water, ethanol, volatile silicone derivatives, and mixtures thereof. The solvents used in such mixtures may be miscible or immiscible with each other. Mousses and aerosol hair sprays can also utilise any of the conventional propellants to deliver the material as a foam (in the case of a mousse) or as a fine, uniform spray (in the case of an aerosol hair spray). Examples of suitable propellants include materials such as trichlorofluoromethane, dichlorodifluoromethane, difluoroethane, dimethylether, propane, n-butane or isobutane. A tonic or hair spray product having a low viscosity may also utilise an emulsifying agent. Examples of suitable emulsifying agents include nonionic, cationic, anionic surfactants, or mixtures thereof. If such an emulsifying agent is used, it is preferably present at a level of from about 0.01% to about 7.5% by weight based on total weight of the composition. The level of propellant can be adjusted as desired but is generally from about 3% to about 30% by weight based on total weight for mousse compositions and from about 15% to about 50% by weight based on total weight for aerosol hair spray compositions.

Suitable spray containers are well known in the art and include conventional, non-aerosol pump sprays i.e., "atomisers", aerosol containers or cans having propellant, as described above, and also pump aerosol containers utilising compressed air as the propellant.

Where the hair care compositions are conditioners and rinses, the carrier can include a wide variety of conditioning materials. Where the hair care compositions are shampoos, the carrier can include, for example, surfactants, suspending agents, and thickeners. Hair styling creams or gels also typically contain a structurant or thickener, typically in an amount of from 0.01% to 10% by weight.

The carrier can be in a wide variety of forms. For example, emulsion carriers, including oil-in-water, water-in-oil, water-in-oil-in-water, and oil-in-water-in-silicone emulsions, are useful herein. These emulsions can cover a broad range of viscosities, e.g., from about 100 cps to about 200,000 cps. These emulsions can also be delivered in the form of sprays using either mechanical pump containers or pressurised aerosol containers using conventional propellants. These carriers can also be delivered in the form of a mousse. Other suitable topical carriers include anhydrous liquid solvents such as oils, alcohols, and silicones (e.g., mineral oil, ethanol, isopropanol, dimethicone, cyclomethicone, and the like); aqueous-based single phase liquid solvents (e.g., hydro-alcoholic solvent systems); and thickened versions of these anhydrous and aqueous-based single phase solvents (e.g., where the viscosity of the solvent has been increased to form a solid or semi-solid by the addition of appropriate gums, resins, waxes, polymers, salts, and the like).

A wide variety of additional components can be employed in hair compostions. Examples include the following:

### Hair Styling Compositions

- hair styling polymers, for hair styling compositions such as hair sprays, gels, and mousses. Hair styling polymers are well known articles of commerce and many such polymers are available commercially which contain moieties which render the polymers cationic, anionic, amphoteric or nonionic in nature. The polymers may be synthetic or naturally derived.

The amount of the hair styling polymer may range from 0.5 to 10%, preferably 0.75 to 6% by weight based on total weight of the composition.

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.

The polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ®ES series available from ISP corporation (esterified copolymers of methyl vinyl ether and maleic anhydride).

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955. Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic hair styling polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Zuviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally-derived hair styling polymers include shellac, alginates, gelatins, pectins, cellulose derivatives and chitosan or salts and derivatives thereof. Commercially available examples include Kytamer® (ex Amerchol) and Amaze® (ex National Starch).

Also suitable for use as optional components in the compositions of the invention are the ionic copolymers described in WO 93/03703, the polysiloxane-grafted polymers disclosed in WO 93/23446, the silicone-containing polycarboxylic acid copolymers described in WO 95/00106 or WO 95/32703, the thermoplastic elastomeric copolymers described in WO 95/01383, WO 95/06078, WO 95/06079 and WO 95/01384, the silicone grafted adhesive polymers disclosed in WO 95/04518 or WO 95/05800, the silicone macro-grafted copolymers taught in WO 96/21417, the silicone macromers of WO 96/32918, the adhesive polymers of WO 98/48770 or WO 98/48771 or WO 98/48772 or WO 98/48776, the graft polymers of WO 98/51261 and the grafted copolymers described in WO 98/51755.

With certain of the above-described polymers it may be necessary to neutralise some acidic groups to promote solubility/dispersibility. Examples of suitable neutralising agents include 2-amino-2- methyl-1, 3-propanediol (AMPD); 2-amino-2-ethyl-1,3-propanediol (AEPD); 2-amino-2-methyl-1-propanol (AMP); 2-amino-1-butanol (AB); monoethanolamine (MEA); diethanolamine (DEA); triethanolamine (TEA); monoisopropanolamine (MIPA); diisopropanol-amine (DIPA); triisopropanolamine (TIPA); and dimethyl stearamine (DMS). A long chain amine neutralising agent such as stearamidopropyl dimethylamine or lauramidopropyl dimethylamine may be employed, as is described in US 4,874,604. Also suitable are inorganic neutralisers, examples of which include sodium hydroxide, potassium hydroxide and borax. Mixtures of any of the above neutralising agents may be used. Amounts of the neutralising agents will range from about 0.001 to about 10% by weight of the total composition.

### Shampoo Compositions

Shampoo compositions preferably comprise one or more cleansing surfactants which are cosmetically acceptable and suitable for topical application to the hair. Further surfactants may be present as emulsifiers.

Suitable cleansing surfactants, are selected from anionic, amphoteric and zwitterionic surfactants, and mixtures thereof. The cleansing surfactant may be the same surfactant as the emulsifier, or may be different.

### Anionic Cleansing Surfactant

Shampoo compositions according to the invention will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alphaolefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl sulpho succinate, ammonium lauryl sulphosuccinate, ammonium lauryl sulphate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n ranges from 1 to 3), ammonium lauryl sulphate and ammonium lauryl ether sulphate(n)EO, (where n ranges from 1 to 3).

The total amount of anionic cleansing surfactant in shampoo compositions of the invention is generally from 5 to 30, preferably from 6 to 20, more preferably from 8 to 16 wt%.

### Co-surfactant

The shampoo composition can optionally include co-surfactants, preferably an amphoteric or zwitterionic surfactant, which can be included in an amount ranging from 0 to about 8, preferably from 1 to 4 wt%.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred co-surfactant is a nonionic surfactant, which can be included in an amount ranging from 0 to 8, preferably from 2 to 5 wt%.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain C₅ to C₂₀ alkylor alkenyl group, G is a saccharide group and n is from 1 to 10.

Other sugar-derived nonionic surfactants which can be included in shampoo compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The shampoo composition can also optionally include one or more cationic co-surfactants included in an amount ranging from 0.01 to 10, more preferably from 0.05 to 5, most preferably from 0.05 to 2 wt%. Useful cationic surfactants are described hereinbelow in relation to conditioner compositions.

The total amount of surfactant (including any co-surfactant, and/or any emulsifier) in shampoo compositions of the invention is generally from 5 to 50, preferably from 5 to 30, more preferably from 10 to 25 wt%.

### Cationic Polymer

A cationic polymer is a preferred ingredient in shampoo compositions of the invention, for enhancing conditioning performance of the shampoo.

The cationic polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

Suitable cationic nitrogen polymers are described in the CTFA Cosmetic Ingredient Directory, 3rd edition

The cationic conditioning polymers can comprise mixtures of monomer units derived from amine- and/or quaternary ammonium-substituted monomer and/or compatible spacer monomers.

Suitable cationic conditioning polymers include, copolymers of 1-vinyl-2-pyrrolidine and 1-vinyl-3-methylimidazolium salt (CTFA name Polyquaternium-16); copolymers of 1-vinyl-2-pyrrolidine and dimethylaminoethyl methacrylate, (CTFA name Polyquaternium-11); cationic diallyl quaternary ammonium-containing polymers in patricular(CTFA Polyquaternium 6 and Polyquaternium 7, mineral acid salts of amino-alkyl esters of homo-and copolymers of unsaturated carboxylic acids as described in U.S. Patent 4,009,256; cationic polyacrylamides(as described in WO95/22311

Cationic polysaccharide polymers suitable for use in compositions of the invention include those with an anhydroglucose residual group, such as a starch or cellulose. Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series). Particularly preferred cationic polymers are JAGUAR C13S, JAGUAR C14, JAGUAR C15, JAGUAR C17 and JAGUAR C16 Jaguar CHT and JAGUAR C162.

The cationic conditioning polymer will generally be present in compositions of the invention at levels of from 0.01 to 5, preferably from 0.05 to 1, more preferably from 0.08 to 0.5 wt%.

### Conditioner Compositions

### Conditioning Surfactant

Conditioner compositions usually comprise one or more conditioning surfactants which are cosmetically acceptable and suitable for topical application to the hair.

Suitable conditioning surfactants are selected from cationic surfactants, used singly or in admixture.

Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula: [N (R₁) (R₂) (R₃) (R₄)]⁺ (X) ⁻
in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

The most preferred cationic surfactants for conditioner compositions of the present invention are monoalkyl quaternary ammonium compounds in which the alkyl chain length is C16 to C22.

Examples of suitable cationic surfactants include quaternary ammonium compounds, particularly trimethyl quaternary compounds.

Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these where the chloride is replaced by halogen, (e.g. , bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

Salts of primary, secondary, and tertiary fatty amines are also suitable cationic surfactants. The alkyl groups of such amines preferably have from 12 to 22 carbon atoms, and can be substituted or unsubstituted.

Particularly useful are amido substituted tertiary fatty amines, in particular tertiary amines having one C₁₂ to C₂₂ alkyl or lakenyl chain. Such amines, useful herein, include stearamidopropyIdimethylamine, stearamidopropyidiethylamine, stearamidoethyldiethylamine, stearamidoethyldimethylamine, palmitamidopropyld imethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachid amidopropyldiethylamine, arachidamidoethyldiethylamine, arachidamidoethyldimethylamine, diethylaminoethylstearamide. Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

hese amines are typically used in combination with an acid to provide the cationic species. The preferred acid useful herein includes L- glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid. Cationic amine surfactants included among those useful in the present invention are disclosed in U.S. Patent 4,275,055 to Nachtigal, et al., issued June 23, 1981.

The molar ratio of protonatable amines to H⁺ from the acid is preferably from about 1:0.3 to 1:1.2, and more preferably from about 1:0.5 to about 1:1.1.

In the conditioners of the invention, the level of cationic surfactant is preferably from 0.01 to 10, more preferably 0.05 to 5, most preferably 0.1 to 2 wt% of the total composition.

The cationic surfactants detailed in this section are also suitable for use in the aspect of the invention wherein a cationic surfactant is intimately mixed with the thermotropic mesogenic material and with oily conditioning material prior to the incorporation of the conditioning material into the final hair conditioning composition

### Fatty Materials

Conditioner compositions of the invention preferably additionally comprise fatty materials. The combined use of fatty materials and cationic surfactants in conditioning compositions is believed to be especially advantageous, because this leads to the formation of a structured phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Representative fatty materials comprise from 8 to 22 carbon atoms, more preferably 16 to 22. Examples of suitable fatty alcohols include cetyl alcohol, stearyl alcohol and mixtures thereof. The use of these materials is also advantageous in that they contribute to the overall conditioning properties of compositions of the invention.
Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.
The level of fatty alcohol material in conditioners of the invention is suitably from 0.01 to 15, preferably from 0.1 to 10, and more preferably from 0.1 to 5 wt%. The weight ratio of cationic surfactant to fatty alcohol is suitably from 10:1 to 1:10, preferably from 4:1 to 1:8, optimally from 1:1 to 1:7, for example 1:3.

### OPTIONAL INGREDIENTS

### Suspending Agents

In a preferred embodiment, the hair treatment composition, especially if it is a shampoo composition, further comprises from 0.1 to 5 wt% of a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

### Conditioning Agents

### Silicone Conditioning Agents

The compositions of the invention can contain, emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 µm, ideally from 0.01 to 1 µm. Silicone emulsions having an average silicone droplet size of ≤ 0.15 µm are generally termed microemulsions.

Examples of suitable pre-formed emulsions include emulsions DC2-1766, DC2-1784, DC-1785 DC-1786 and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone", Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones). Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant. Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

With some shampoos it is particularly preferred to use a combination of amino and non amino functional silicones

The total amount of silicone is preferably from 0.01 to 10 %wt of the total composition more preferably from 0.3 to 5, most preferably 0.5 to 3 wt% is a suitable level.

### General ingredients suitable for all product forms:

- sunscreening agents such as 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof.
- anti-dandruff actives such as zinc pyrithione, piroctone olamine, selenium disulphide, sulphur, coal tar, and the like.
- carboxylic acid polymer thickeners for hair shampoo and conditioner compositions. These crosslinked polymers contain one or more monomers derived from acrylic acid, substituted acrylic acids, and salts and esters of these acrylic acids and the substituted acrylic acids, wherein the crosslinking agent contains two or more carbon-carbon double bonds and derived from a polyhydric alcohol. Examples of carboxylic acid polymer thickeners useful herein are those selected from the group consisting of carbomers, acrylates/C₁₀-C₃₀ alkyl acrylate crosspolymers, and mixtures thereof. Compositions of the present invention can comprise from about 0.025% to about 1%, more preferably from about 0.05% to about 0.75% and most preferably from about 0.10% to about 0.50% of the carboxylic acid polymer thickeners, by weight based on total weight of the composition.
- emulsifiers for emulsifying the various carrier components of the compositions of the invention. Suitable emulsifier types include polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate,Polysorbate 60, glyceryl stearate, PEG-100 stearate, and mixtures thereof. The emulsifiers can be used individually or as a mixture of two or more and can comprise from about 0.1% to about 10%, more preferably from about 1% to about 7%, and most preferably from about 1% to about 5%, by weight based on total weight of the composition.
- vitamins and derivatives thereof (e.g., ascorbic acid, vitamin E, tocopheryl acetate, retinoic acid, retinol, retinoids, and the like).
- preservatives, antioxidants, chelators and sequestrants; and aesthetic components such as fragrances, colourings, hair nutrients and essential oils.

The method of the invention comprises applying a compound of formula I as described herein to the hair. The compound is preferably in the form of a composition of the invention when it is applied to the hair, although other product forms may also be used, such as for example a simple solution of the compound.

### (ii) Non-silicone Oily Conditioning Components

Compositions according to the present invention may also comprise a dispersed, non-volatile, water-insoluble oily conditioning agent.

By "insoluble" is meant that the material is not soluble in water (distilled or equivalent) at a concentration of 0.1% (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C₂-C₆ alkenyl monomers.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and triesters of glycerol and long chain carboxylic acids such as C₁-C₂₂ carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 wt%.

The compositions of this invention preferably contain no more than 3 wt% of a styling polymer, more preferably less than 1% of a styling polymer, preferably contain less than 0.1% by weight a styling polymer, and optimally are free of styling polymer.

In hair treatment compositions containing a conditioning agent, it is preferred that a cationic polymer also be present.

### Adjuncts

The compositions of the present invention may also contain adjuncts suitable for hair care. Generally such ingredients are included individually at a level of up to 2, preferably up to 1 wt% of the total composition.
Suitable hair care adjuncts, include amino acids, sugars and ceramides.

The method of the invention may involve the conventional steps in hair treatment methods. For example, the method may involve shampooing and/or conditioning (either as part of shampooing or in a separate process step) and/or styling the hair.

The following non-limiting Examples further illustrate the preferred embodiments of the invention. All percentages referred to in the examples and throughout this specification are by weight based on total weight unless otherwise indicated.

### EXAMPLES

### Examples 1 to 10 - Compositions of the Invention

The following are examples of compositions of the invention. The materials in the examples include the following:

| **Material** | **Chemical Name** | **Supplier** |
|---|---|---|
| Silicone emulsion X2 1787^{™} | | Dow Corning |
| VOLPO CS 50^{™} | | Croda Chemcials |
| Silcone DC 200/DC 24 S^{™} | | Dow Corning |
| Silwet L7602/L-720^{™} | | Union Carbide |
| CAP 40^{™} | | Calor Gas |
| Carbopol 980^{™} | | BF Goodrich |
| Jaguar HP-105^{™} | | Rhodia |
| Silicone Fluid 245^{™} | | Dow Corning |
| Gafquat 734(trademark) | Polyquaternium-11 | ISP |
| PVP K-120 | | ISP |
| Amphomer 284910 | | National starch |
| Ethanol is SD Alcohol 40-B (92% active) | | |
| Compound 1 | 2,5dihydroxy benzoic acid | |
| Compound 2 | 3,4dihydroxy benzoic acid | |
| Compound 3 | 3, 5dihydroxy benzoic acid | |
| Compound 4 | 3, 4 dihydroxy benzaldyehde | |

### Example 1

A styling mousse is formulated as follows:

| **Material** | **% in product (w/w)** |
|---|---|
| Silicone Emulsion X2 1787 | 1.2 |
| Compound 1,2,3, or 4 | 1.5 |
| Gafquat 734 | 2.0 |
| VOLPO CS 50 | 0.3 |
| Sepicide LD | 0.4 |
| Cremophor RH410 | 0.2 |
| Ethanol | 7.5 |
| CAP 40 | 8.0 |
| Perfume | 0.2 |
| Water | to 100% |

### Example 2

A hairspray is formulated as follows:

| **Material** | **% in product (w/w)** |
|---|---|
| Compound 1,2,3, or 4 | 1.3 |
| Amphomer 284910 | 3.0 |
| Silicone DC200 | 0.09 |
| Silwet L7602 | 0.09 |
| CAP 40 | 35.0 |
| Ethanol | 60.0 |
| Perfume | 0.10 |
| Water | to 100% |

### Example 3

A pump spray is formulated as follows:

| **Material** | **% w/w** |
|---|---|
| Amphomer | 3.5 |
| Silwet L-720 | 0.3 |
| Silicone DC24S | 0.15 |
| Fragrance | 0.3 |
| Water | to 100% |
| Ethanol | 60.0 |
| Compound 1,2,3, or 4 | 1.0 |

### Example 4

A styling gel is formulated as follows:

| **Material** | **% w/w** |
|---|---|
| Compound 1,2,3, or 4 | 0.9 |
| PVP | 3.8 |
| Carbopol 980 | 0.4 |
| Water | to 100% |
| Sepicide LD | 0.4 |
| Sodium hydroxide (8% 2M) | 0.1 |
| Ethanol | 10.0 |
| Cremaphor RH410 | 0.4 |
| Jaguar HP-105 | 0.2 |
| Perfume | 0.15 |

### Example 5

A 55% voc propelled aerosol composition is formulated as follows:

| **Material** | **% w/w** |
|---|---|
| Compound 1,2,3, or 4 | 0.6 |
| Amphomer | 3.0 |
| Silicone Fluid 245 | 0.20 |
| Fragrance | 0.32 |
| Ethanol | 19.53 |
| Dimethyl ether | 35.00 |
| Sodium benzoate | 0.26 |
| Cyclohexylamine | 0.21 |
| Water | to 100% |

### Example 6

A 55% voc pump hairspray composition is formulated as follows:

| **Material** | **% w/w** |
|---|---|
| Compound 1,2,3, or 4 | 0.6 |
| Amphomer | 3.0 |
| Cyclopentasiloxane (99% active) | 0.15 |
| Benzophenone 4 | 0.0001 |
| Fragrance | 0.25 |
| Ethanol | 58.00 |
| Water | to 100% |

### Example 7

The following is an example of a shampoo composition according to the invention:

| **Ingredient** | |
|---|---|
| **Chemical Name** | **Active weight %** |
| SLES 2EO | 14 |
| Cocoamidopropylbetaine | 2 |
| Guar hydroxypropyltrimonium chloride | 0.1 |
| Dimethiconol | 1 |
| Mica + titanium dioxide | 0.2 |
| Sodium benzoate | 0.5 |
| Water | to 100 |
| Crosslinked polyacrylic acid | 0.4 |
| Compound 1,2,3, or 4 | 1 |

### Example 8

The following is an example of a hair conditioning composition according to the invention:

| **Ingredient** | **Trade name** | **Supplier** | **Function** | **%w/w** |
|---|---|---|---|---|
| Deionised water | | | solvent | q.s. to 100 |
| Compound 1, 2, 3 or 4 | | | | 1 |
| Behenyl trimethyl ammonium chloride | Genamin KDM-P | Clairant | cationic surfactant | 1 |
| Behenyl alcohol | | | fatty alcohol | 5 |
| Silicone emulsion | DC2-1784 | Dow Corning | conditioning ingredient | 2 |
| Disodium EDTA | | | sequesterant | 0.1 |
| Methyl paraben | Nipagin | Nipa Lab | preservative | 0.2 |
| Perfume | | | fragrance | 0.5 |

### Example 9

### Conditioner

| **Ingredient** | **Trade name** | **Supplier** | **Function** | **%w/w** |
|---|---|---|---|---|
| Deionised water | | | solvent | q.s. to 100 |
| Compound 1,2,3 3 or 4 | | | | 1 |
| Behenyl trimethyl ammonium chloride | Genamin KDM-P | Clairant | cationic surfactant | 1 |
| Behenyl alcohol | | | fatty alcohol | 5 |
| Disodium EDTA | | | sequesterant | 0.1 |
| Methyl paraben | Nipagin | Nipa Lab | preservative | 0.2 |
| Perfume | | | fragrance | 0.5 |

### Example 10

| **Ingredient** | **Wt%** |
|---|---|
| Volvo CP | 0.1 |
| Compound 1,2,3,4 | 1 |
| Silicone emulsion | 1.2 |
| Perfume | 0.32 |
| Water | to 100 |

### Example 11

Three solutions were formulated each containing 1% by weight of either 2,5 dihydroxybenzoic acid, 3,4 dihydroxybenzoic acid or 3,5 dihydroxybenzoic acid. Hair curls were made up using perming rods and 30cm length hair. The curls were placed in the solution for 1 hour, rinsed thoroughly with distilled water and dried for 30 minutes. The hair was removed from the curling rod and the length of the curl measured. The hair was hung in high humidity environmental chamber (90%RH, 30°C) with fan agitation for 1 minute and measured again.

| | **Styling compound** | **Wt%** | **Solvent** | Curl dropout % |
|---|---|---|---|---|
| Negative Control | Water (no additional compound) | 100% | Water | 64 |
| Test Compound | 2,5 dihydroxybenzoic acid | 1% | Water | 17.1 |
| Test Compound | 3,4 dihydroxybenzoic acid | 1% | Water | 29 |
| Test Compound | 3,5 dihydroxybenzoic acid | | Water | 26 |
| Positive Control* | Commercial Hairspray | n/a | n/a | 45.4 |

| | | | | |
|---|---|---|---|---|
| *Spray (John Freda Frizzease UK, 2000) applied after curl was treated and dried as negative control. | | | | |

## Claims

1. A method of styling hair comprising the step of applying to the hair a non-colourant treatment composition comprising 0.1 to 3 wt% of the total composition of a styling compound of formula 1 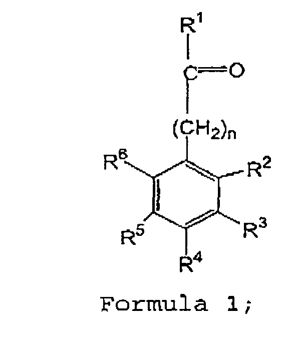
wherein n is equal to 0,
R¹ is a H , OH, alkyl, alkoxide group or salts thereof; R², R³, R⁴, R⁵ and R⁶ are independently selected from OH, H, C₁-C₄ alkyl groups or mixtures thereof such that the total number of OH groups of R² , R³ , R⁴, R and R is 2, such that when R² is an OH group R⁵ is also an OH group; and
in which the composition comprises less than 2 wt% of a starch or starch derivative.

2. A method of styling hair comprising the step of applying to the hair a hair treatment composition comprising 0.1 to 3 wt% of the total composition of a styling compound of formula 1 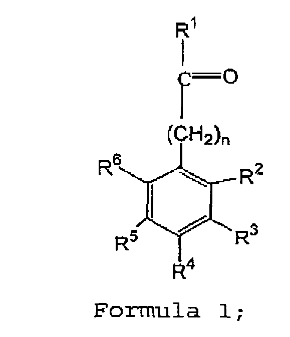 wherein n is equal to 0,
R¹ is a H, OH, alkyl, alkoxide group or salts thereof;
R², R³, R⁴, R⁵ and R⁶ are independently selected from OH, H, C₁-C₄ alkyl groups or mixtures thereof such that the total number of OH groups of R², R³ , R⁴ , R⁵ and R is 2, such that when R² is an OH group R⁵ is also an OH group;
in which the composition is free of compounds capable of reacting to form covalent bonds below 70°C with the compound of formula 1, when both compounds are applied to the hair.

3. A method of styling hair according to any preceding claim in which R², R³, R⁴, R⁵ and R⁶ are either OH or H.

4. A method of styling hair according to any preceding claim in which the styling compound is selected from the following formulae:

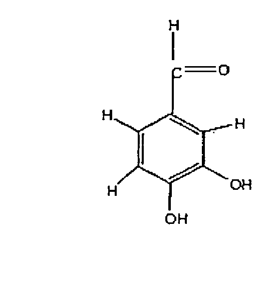

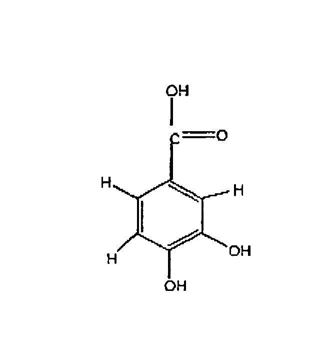

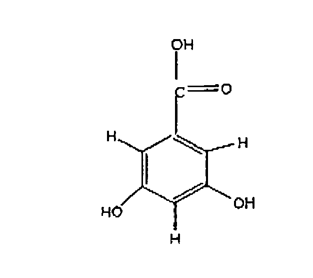

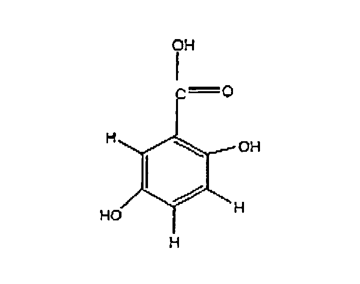

5. A method according to any preceding claim in which the composition is applied or a leave on product.

6. A method according to any one of claims 1 to 4 in which the composition is in the form of a rinse off product.

7. A method according to any preceding claim in which the composition is in the form of a shampoo, conditioner, gel or mousse.

8. A method according to any preceding claim in which the composition further comprises a buffer or pH adjuster.

9. Use of a compound of formula 1 for styling hair 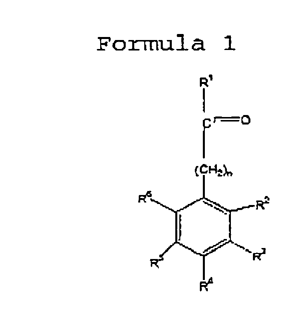 wherein n is equal to 0,
R¹ is a H , OH, alkyl, alkoxide group or salts thereof;
R², R³, R⁴, R⁵ and R⁶ are independently selected from OH, H, C₁-C₄ alkyl groups or mixtures thereof such that the total number of OH groups of R², R³ R⁴, R⁵ and R⁶ is 2 and when R² is an OH group. R⁵ is also an OH group.

## Patentansprüche

1. Haarstylingverfahren, umfassend den Schritt der Auftragung einer nicht färbenden Behandlungszusammensetzung auf das Haar, die 0,1 bis 3 Gew.-% der Gesamtzusammensetzung einer Formgebungsverbindung der Formel 1 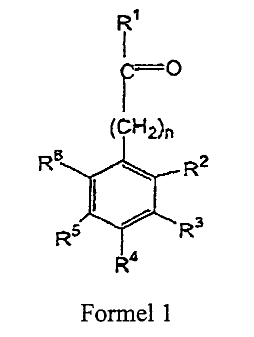
umfaßt, worin n gleich 0 ist,
R¹ H, eine OH-, Alkyl-, Alkoxidgruppe oder Salze davon ist;
R², R³, R⁴, R⁵ und R⁶ unabhängig aus H, OH-, C₁-C₄-Alkylgruppen oder Gemischen davon ausgewählt sind, so daß die Gesamtanzahl an OH-Gruppen von R², R³, R⁴, R⁵ und R⁶ 2 ist, so daß, wenn R² eine OH-Gruppe ist, R⁵ auch eine OH-Gruppe ist; und
worin die Zusammensetzung weniger als 2 Gew.-% einer Stärke oder eines Stärkederivats umfaßt.

2. Haarstylingverfahren, umfassend den Schritt der Auftragung einer Haarbehandlungszusammensetzung auf das Haar, die 0,1 bis 3 Gew.-% der Gesamtzusammensetzung einer Formgebungsverbindung der Formel 1 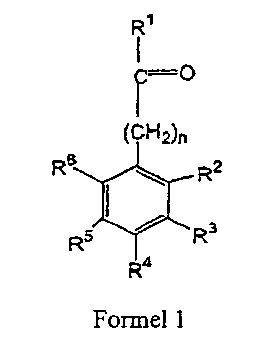
umfaßt, worin n gleich 0 ist,
R¹ H, eine OH-, Alkyl-, Alkoxidgruppe oder Salze davon ist;
R², R³, R⁴, R⁵ und R⁶ unabhängig aus H, OH-, C₁-C₄-Alkylgruppen oder Gemischen davon ausgewählt sind, so daß die Gesamtanzahl an OH-Gruppen von R², R³, R⁴, R⁵ und R⁶ 2 ist, so daß, wenn R² eine OH-Gruppe ist, R⁵ auch eine OH-Gruppe ist;
worin die Zusammensetzung frei von Verbindungen ist, die unter 70 °C unter Bildung kovalenter Bindungen mit der Verbindung der Formel 1 reagieren können, wenn beide Verbindungen auf das Haar aufgetragen werden.

3. Haarstylingverfahren nach einem der vorhergehenden Ansprüche, worin R², R³, R⁴, R⁵ und R⁶ entweder OH oder H sind.

4. Haarstylingverfahren nach einem der vorhergehenden Ansprüche, worin die Formgebungszusammensetzung aus den folgenden Formeln ausgewählt ist:

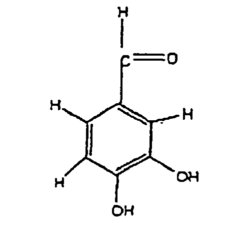

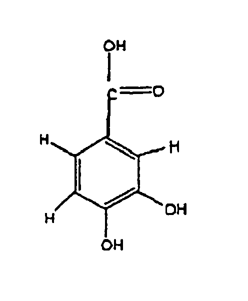

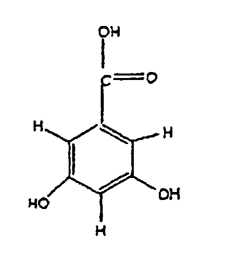

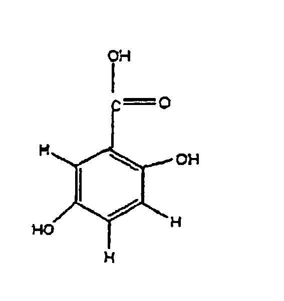

5. Verfahren nach einem der vorhergehenden Ansprüche, worin die Zusammensetzung als ein Produkt zum Verbleib aufgetragen wird.

6. Verfahren nach einem der Ansprüche 1 bis 4, worin die Zusammensetzung in Form eines Produktes zum Ausspülen vorliegt.

7. Verfahren nach einem der vorhergehenden Ansprüche, worin die Zusammensetzung in Form eines Shampoos, eines Pflegemittels, eines Gels oder einer Mousse vorliegt.

8. Verfahren nach einem der vorhergehenden Ansprüche, worin die Zusammensetzung ferner einen Puffer oder pH-Einsteller umfaßt.

9. Verwendung einer Verbindung der Formel 1 zum Stylen des Haars 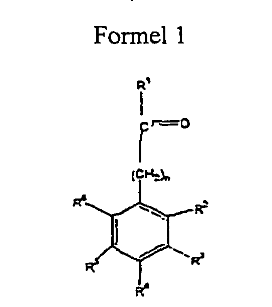 worin n gleich 0 ist,
R¹ H, eine OH-, Alkyl-, Alkoxidgruppe oder Salze davon ist;
R², R³, R⁴, R⁵ und R⁶ unabhängig aus H, OH-, C₁-C₄-Alkylgruppen oder Gemischen davon ausgewählt sind, so daß die Gesamtanzahl an OH-Gruppen von R², R³, R⁴, R⁵ und R⁶ 2 ist und wenn R² eine OH-Gruppe ist, R⁵ auch eine OH-Gruppe ist.

## Revendications

1. Procédé pour coiffer les cheveux comprenant l'étape consistant à appliquer aux cheveux une composition de traitement capillaire non colorante comprenant de 0,1 à 3 % en poids d'un composé coiffant de formule 1 : 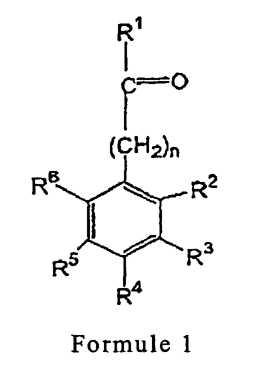
dans laquelle n est égal à 0 ;
R¹ est un H, OH, alkyle, un groupe alkoxyde ou des sels de ceux-ci ;
R², R³, R⁴, R⁵ et R⁶ sont indépendamment choisis à partir de OH, H, les groupes alkyles en C₁ - C₄ ou les mélanges de ceux-ci, de telle sorte que le nombre total de groupes OH de R², R³, R⁴, R⁵ et R⁶ soit 2, de telle sorte que lorsque R² est un groupe OH, R⁵ est également un groupe OH ; et
dans laquelle soit la composition comprend moins de 2 % en poids d'un amidon ou d'un dérivé d'amidon.

2. Procédé pour coiffer les cheveux comprenant l'étape consistant à appliquer aux cheveux une composition de traitement capillaire comprenant de 0,1 à 3 % en poids, sur la base totale de la composition, d'un composé coiffant de formule 1 : 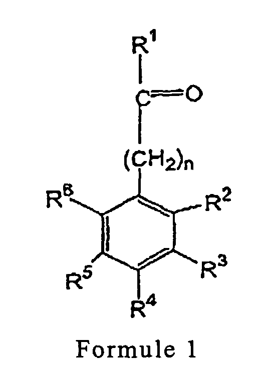
dans laquelle n est égal à 0 ;
R¹ est un groupe H, OH, alkyle, alkoxyde, ou des sels de ceux-ci ;
R², R³, R⁴, R⁵ et R⁶ sont indépendamment choisis parmi OH, H, les groupes alkyles en C₁- C₄ ou des mélanges de ceux-ci, de telle sorte que le nombre total de groupes OH de R², R³, R⁴, R⁵ et R⁶ soit 2, de telle sorte que lorsque 2 est un groupe OH, R5 est également un groupe OH ;
dans laquelle la composition est exempte de composés capables de réagir pour former des liaisons covalentes à une température inférieure à 70°C dans le composé de formule I, lorsque les deux composés sont appliqués aux cheveux.

3. Procédé pour coiffer les cheveux selon l'une quelconque des revendications précédentes, dans lequel R², R³, R⁴, R⁵ et R⁶ sont soit OH, soit H.

4. Procédé pour coiffer les cheveux selon l'une quelconque des revendications précédentes, dans lequel le composé coiffant est choisi parmi les formules suivantes :

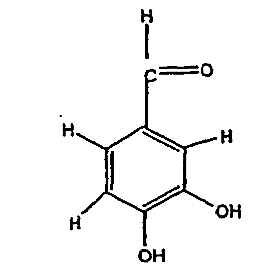

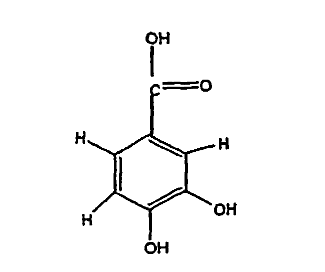

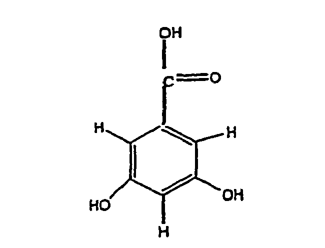

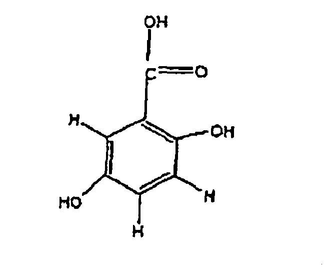

5. Procédé selon l'une quelconque des revendications précédentes, dans lequel la composition est appliquée en tant que produit sans rinçage.

6. Procédé selon l'une quelconque des revendications 1 à 4, dans lequel la composition est sous la forme d'un produit à rincer.

7. Procédé selon l'une quelconque des revendications précédentes, dans lequel la composition est sous la forme d'un shampoing, d'un conditionneur, d'un gel ou d'une mousse.

8. Procédé selon l'une quelconque des revendications précédentes, dans lequel la composition comprend un tampon ou un ajusteur de pH.

9. Utilisation d'un composé de formule 1 pour coiffer les cheveux : 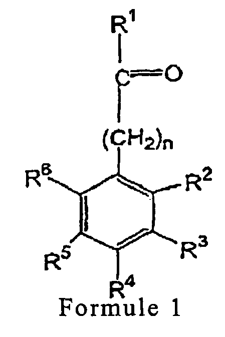
dans laquelle n est égal à 0 ;
R¹ est un H, OH, alkyle, un groupe alkoxyde ou des sels de ceux-ci ;
R² R³, R⁴, R⁵ et R⁶ sont indépendamment choisis à partir de OH, H, les groupes alkyles en C₁ - C₄ ou les mélanges de ceux-ci, de telle sorte que le nombre total de groupes OH de R², R³, R⁴, R⁵ et R⁶ soit 2, de telle sorte que lorsque R² est un groupe OH, R⁵ est également un groupe OH
